(19) Europäisches Patentamt
European Patent Office
Office européen des brevets

(11) EP 0 658 100 B1

(12) **EUROPEAN PATENT SPECIFICATION**

(45) Date of publication and mention of the grant of the patent:
**14.07.1999 Bulletin 1999/28**

(21) Application number: **93917739.0**

(22) Date of filing: **03.08.1993**

(51) Int. Cl.$^6$: **A61K 7/50**, A61K 7/48

(86) International application number:
**PCT/EP93/02072**

(87) International publication number:
**WO 94/03152 (17.02.1994 Gazette 1994/05)**

(54) **DETERGENT COMPOSITION**
WASCHMITTEL
COMPOSITION DE DETERGENT

(84) Designated Contracting States:
**CH DE ES FR GB IT LI NL SE**

(30) Priority: **07.08.1992 GB 9216758**

(43) Date of publication of application:
**21.06.1995 Bulletin 1995/25**

(73) Proprietors:
- **UNILEVER PLC**
  **London EC4P 4BQ (GB)**
  Designated Contracting States:
  **GB**
- **UNILEVER N.V.**
  **3013 AL Rotterdam (NL)**
  Designated Contracting States:
  **CH DE ES FR IT LI NL SE**

(72) Inventor:
**HELLIWELL, John Fielden,**
**9 Crossley Drive**
**Wirral, Merseyside L60 9JA (GB)**

(74) Representative: **Bryant, Tracey**
**Unilever PLC**
**Patent Division**
**Colworth House**
**Sharnbrook**
**Bedford MK44 1LQ (GB)**

(56) References cited:
**EP-A- 0 400 976**
**EP-A- 0 432 951**
**EP-A- 0 463 780**
**EP-A- 0 468 721**
**GB-A- 2 236 321**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 658 100 B1

## Description

This invention relates to detergent compositions in liquid or gel form suitable for personal washing and having a specified minimum viscosity.

There are a variety of commercially available detergent products in liquid form which are intended for personal washing. Shampoos intended for hair washing are customarily formulated so as to have a viscosity which does not substantially exceed 5,000 centipoise at a shear rate of 10 $sec^{-1}$. This enables such compositions to be worked into the hair of the user without undue difficulty.

By contrast some products intended for washing skin are formulated with a rather higher viscosity so that they will not run off too quickly when applied to the skin of the user. Shower gels are a typical form of detergent product frequently formulated to have relatively high viscosity.

It is known to include silicone in shampoo compositions as a hair conditioning aid. A difficulty however is that the emulsified droplets of silicone oil tend to come out of suspension and form a separate layer at the top of the liquid phase. It is known to include cationic polymer in shampoo compositions together with the silicone oil. The cationic polymer acts as another conditioning aid. Nevertheless in order to obtain a shampoo composition which is stable on storage some additional measure is necessary. One possibility is to include a diester of a dihydric alcohol such as ethyleneglycol distearate which functions to structure the emulsion and inhibit the separation of silicone oil. Another approach is to incorporate compounds which are secondary alcohols having 2 alkyl chains extending from a central moiety which includes the secondary hydroxyl group.

These secondary alcohols and the diesters can be classified as compounds which incorporate 2 alkyl groups with a total of at least 20 carbon atoms between them. Other materials which have been proposed for this purpose are acyl derivatives in which the acyl groups contain at least 16 carbon atoms, amine oxide with alkyl chains of at least 16 carbon atoms, and Scleroglucan gums.

Specific compositions of the general type described above are found in EP-A-432951 (Unilever:1989). EP-A-432951 describes shampoo formulations comprising sodium lauryl ether sulphate and betaine surfactants, Jaguar cationic polymer and a silicone conditioner.

We have now found that when a detergent composition containing silicone and cationic polymer is formulated with a higher viscosity than is customary for a shampoo, the storage stability improves very remarkably and effectively ceases to be a problem. This is especially true when the average droplet size of the silicone is less than 2 μm in diameter.

Therefore, according to the present invention there is provided a detergent composition in the form of an aqueous liquid or gel, a substantial part of which is in the rod-micellar phase, containing:

5 to 50% by weight of non-soap detergent,
0.01 to 5% by weight of cationic polymer, and
2.0 to 15% by weight of silicone,
where the detergent composition has a viscosity of at least 6.0 Pa.s (6000cP) at a shear rate of 10 $sec^{-1}$, the composition not including more than 0.5% by weight of structuring compounds in the form of esters or alcohols incorporating two alkyl or acyl groups with more than 20 carbon atoms in total, nor more than 0.5% by weight of Scleroglucan gums. Preferably the viscosity is at least 7.0 Pa.s (7000cP) at 10 $sec^{-1}$ shear rate.

Further the present invention also provides a shampoo in the form of an aqueous liquid or gel, a substantial part of which is in the rod-micellar phase, containing:

5 to 50% by weight of non-soap detergent,
0.01 to 5% by weight of cationic polymer, and
0.5 to 15% by weight of silicone,
where the detergent composition has a viscosity of at least 6.0 Pa.s (6000cP) at a shear rate of 10 $sec^{-1}$, the composition not including more than 0.5% by weight of structuring compounds in the form of esters on alcohols incorporating two alkyl or acyl groups with more than 20 carbon atoms in total, nor more than 0.5% by weight of Scleroglucan gums.

While it might be considered that a high viscosity product such as a shower gel would show a reduced tendency cream. We have observed that the addition of non-silicone oils to conventional shower gel formulations results in a destruction of the rod-micells and consequently to creaming of the added oil. Surprisingly, this does not occur where the oil is a silicone.

In this invention it is not necessary to include such structuring compounds as esters and alcohols having two alkyl or acyl groups with more than 20 carbon atoms in total, acyl derivatives in which the acyl groups each contain at

least 16 carbon atoms, Scleroglucan gums and amine oxides incorporating alkyl groups with at least 16 carbon atoms. Such compounds are then preferably absent or if present, are included in an amount less than 0.5%.

The total amount of detergent in the composition preferably does not exceed 25% by weight, and may well not exceed 20% by weight. The amount of detergent may well be at least 8% or even at least 10% of the composition.

The detergent may be a variety of materials from the well known classes of non-soap anionic, nonionic, amphoteric and zwitterionic surfactants.

Preferably the detergent comprises anionic detergent either alone or accompanied by a zwitterionic detergent, for instance the anionic detergent may be 30 to 100%, preferably at least 50%, preferably not over 80% of the detergent present while the zwitterionic detergent is 0 to 70%, preferably at least 20%, preferably not over 50% of the detergent present.

One preferred anionic detergent is fatty acyl isethionate of formula:

$$RCO_2CH_2CH_2SO_3M$$

where R is an alkyl or alkenyl group of 7 to 21 carbon atoms and M is a solubilising cation such as sodium, potassium, ammonium or substituted ammonium. Preferably at least three quarters of the RCO groups have 12 to 18 carbon atoms and may be derived from coconut.

Another preferred anionic detergent is alkyl ether sulphate of formula:

$$RO(CH_2CH_2O)_nSO_3M$$

where R is an alkyl group of 8 to 22 carbon atoms,
n ranges from 0.5 to 10 especially 1.5 to 8, and
M is a solubilising cation as before.

Other possible anionic detergents include alkyl glyceryl ether sulphate, sulphosuccinates, taurates, sarcosinates, sulphoacetates, alkyl phosphates and acyl lactates. Sulphosuccinates may be monoalkyl sulphosuccinates having the formula:
$R^5O_2CCH_2CH(SO_3M)CO_2M$; and amido-MEA sulphosuccinates of the formula: $R^5CONHCH_2CH_2O_2CCH_2CH(SO_3M)CO_2M$; wherein $R^5$ ranges from $C_8$-$C_{20}$ alkyl, preferably $C_{12}$-$C_{15}$ alkyl and M is a solubilising cation.

Sarcosinates are generally indicated by the formula: $R^5CON(CH_3)CH_2CO_2M$, wherein R ranges from $C_8$-$C_{20}$ alkyl, preferably $C_{12}$-$C_{15}$ alkyl and M is a solubilising cation.

Taurates are generally identified by the formula: $R^5CONR^6CH_2CH_2SO_3M$, wherein $R^5$ ranges from $C_8$-$C_{20}$ alkyl, preferably $C_{12}$-$C_{15}$ alkyl, $R^6$ ranges from $C_1$-$C_4$ alkyl, and M is a solubilising cation.

The anionic detergent included in the composition will generally be selected to avoid harsh detergent such as primary alkane sulphonate or alkyl benzene sulphonate.

The amount, if any, of these is preferably less than 3% of the detergents present.

Suitable switterionic detergents have a hydrophilic head group which contains both a quaternary nitrogen atom and at least one acid group which may be a carboxylic or a sulphonic acid group. Such detergents should generally include an alkyl or alkenyl group of 7 to 18 carbon atoms. They will usually comply with an overall structural formula

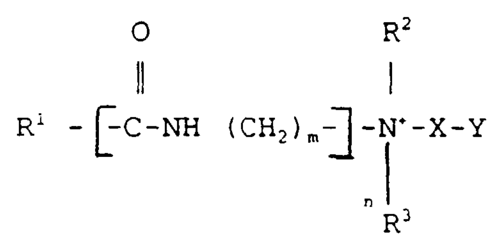

where $R^1$ is alkyl or alkenyl of 7 to 18 carbon atoms
$R^2$ and $R^3$ are each independently alkyl, hydroxyalkyl or carboxyalkyl of 1 to 3 carbon atoms
m is 2 to 4
n is 0 or 1

X is alkylene of 1 to 3 carbon atoms optionally substituted with hydroxyl, and
Y is $-CO_2^-$ or $-SO_3^-$

Zwitterionic detergents within the above general formula include simple betaines of formula:

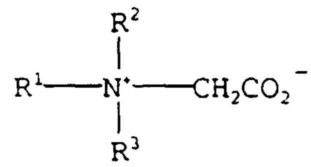

and amido betaines of formula:

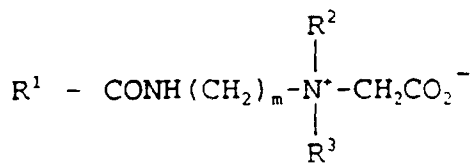

where m is 2 or 3.

In both formulae $R^1$, $R^2$ and $R^3$ are as defined previously. $R^1$ may in particular be a mixture of $C_{12}$ and $C_{14}$ alkyl groups derived from coconut so that at least half, preferably at least three quarters of the groups $R^1$ have 10 to 14 carbon atoms. $R^2$ and $R^3$ are preferably methyl.

A further possibility is that the zwitterionic detergent is a sulphobetaine of formula:

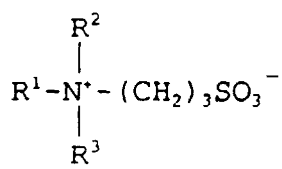

or

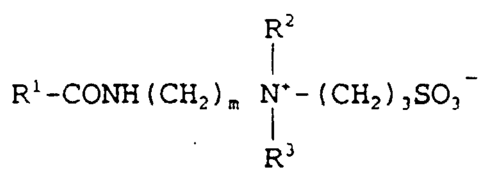

where m is 2 or 3, or variants of these in which $-(CH_2)_3SO_3^-$ is replaced by

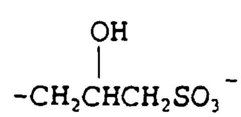

$R^1$, $R^2$ and $R^3$ in these formulae are as defined previously.

Other anionic detergents beside acyl isethionate may be present notably in quantities from 10 to 50% of the detergent mixture. Anionic detergent which is particularly envisaged is alkyl ether sulphate of the formula:

$$R^4O(CH_2CH_2O)_t\ SO_3M$$

where $R^4$ is alkyl or alkenyl of 8 to 18 carbon atoms, especially 11 to 15 carbon atoms, t has an average value of at least 2.0 and M is a solubilising cation such as sodium, potassium, ammonium or substituted ammonium. Preferably t has an average value of 3 or more.

Alkanolamide detergents are preferably included at only a low level, if at all, since they have been found to reduce mildness. Preferably they are restricted to not more than 5% by weight of the detergent mixture. Even better is to exclude alkanolamides and the harsh anionics, alkyl benzene sulphonate and primary alkane sulphonate completely. It is also preferred that amine oxide is not more than 5% by weight of the detergent mixture, since this has been found to reduce lather quality.

In particularly preferred embodiments of the present invention, the surfactant system comprises the combination of a zwitterionic detergent and one or both of an isethionate or an alkyl ether sulphate. As mentioned above, the zwitterionic detergent is preferably a betaine, be that a simple betaine, amido betaine or sulphobetaine.

Preferred ratios being:

0.05-5:1 of alkyl ether sulphate : betaine, and,
0.05-5:1 of cocoyl isethionate : betaine,
where only a single pair of the abovementioned surfactants are present.

Where all three surfactant components are present the ratio of the total of the alkyl ether sulphate and the isetionate will generally account for 25-75% of the surfactant present.

The amount of cationic polymer is preferably at least 0.05% by weight of the whole composition. It may well not exceed 3% or even 2% of the composition.

Various cationic polymers may be used. Examples of cationic polymers include the cationic cellulose ethers described in US Patents Nos. 3816616 and 4272515 and which are available commercially from Union Carbide Corp. under the trade mark POLYMER JR. Other suitable materials are the cationic polygalactomannan gum derivatives described in US Patent No. 4298494 which are commercially available under the trade mark JAGUAR from Celanese-Stein Hall. An example of a suitable material is the hydroxypropyltrimethylammonium derivative of guar gum of the formula:

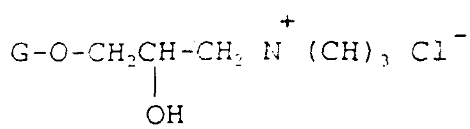

where G represents guar gum. Such a material is available under the name JAGUAR C-13-S. This material also has the CTFA designation Guar Hydroxypropyltrimonium Chloride. In JAGUAR C-13-S the degree of substitution of the cationic groups is about 0.13. Another possible material is that known as JAGUAR C-17 which is similar to JAGUAR C-13-S but has a higher degree of substitution of cationic groups of about 0.25-0.31. A further example of a guar derivative is the nydroxypropylated cationic guar derivative known as JAGUAR C-16 which as well as containing the above cationic quaternary ammonium groups also contain hydroxypropyl ($-CH_2CH(OH)CH_3$) substituent groups. In JAGUAR C-16 the degree of substitution of the cationic groups is 0.11-0.16 and the moles of substitution of hydroxypropyl groups is 0.8-1.1.

A surprising feature of the present invention is that stable depositing compositions can be made with both the cationic cellulose ethers (such as POLYMER JR) and the cationic polygalactomannan gums (such as JAGUAR). While JAGUAR is the more commonly used cationic polymer in the art, polymer JR is less likely to be contaminated with substances which lend a colour to the resulting compositions.

Other cationic polymers include cationic polyamide polymers such as the low molecular weight adipic

acid/diethylene-triamine polyamide and the copolymers of vinylpyrrolidone and dimethylaminoethyl methacrylate quaternised with dimethyl sulphate (Gafquat 755, GAF Corporation) described in US Patent No. 4080310; the graft cationic copolymer containing N-vinylpyrrolidone, dimethylaminoethyl methacrylate and polyethylene glycol described in US Patent No. 4048301; the mineral acid salts of the amino-alkyl esters of homo- and copolymers or unsaturated carboxylic acids having from 3 to 5 carbon atoms described in US Patent No. 4009256; and the polymers of etherified starch described in US Patent No. 3186911.

The high molecular weight cationic polymers are sold under the trade mark MERQUAT by Merck & Co., Inc. Representative ones are Merquat 100, a highly charged cationic dimethyldiallylammonium chloride homopolymer, and Merquat (TM) 550, a highly charged cationic copolymer prepared with dimethyldiallylammonium chloride and acrylamide. These materials are designated in the CTFA dictionary as Quaternium-40 and Quaternium-41, respectively.

It is preferred that the amount of silicone is at least 2% by weight of the composition and preferably at least 3% or 3.5%. The amount will generally not exceed 10% or even 8%.

Silicone may be an involatile silicone oil or a relatively more volatile silicone. An involatile silicone oil will generally have a viscosity of at least 5 centistokes at 25°C, e.g. 10 to 100,000 centistokes. In fact, involatile silicones are highly preferred for the invention.

Suitable silicones include polyalkyl or polyaryl siloxanes with the following structure:

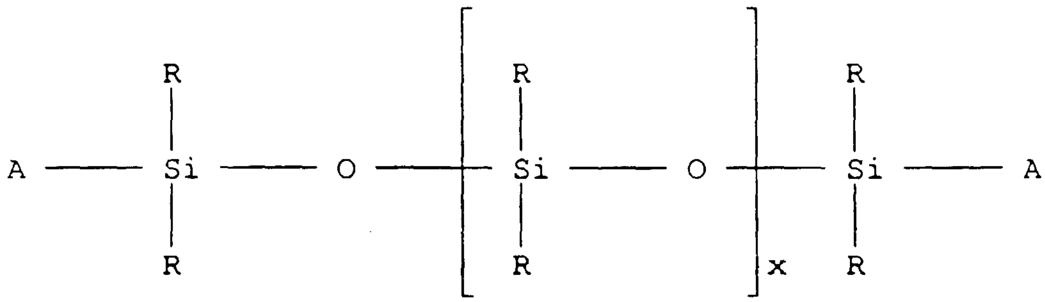

wherein R is alkyl or aryl, x is an integer from about 100 to about 2,400 and A represents groups which block the ends of the silicone chains. Suitable A groups include methyl, methoxy, ethoxy, propoxy, and aryloxy. The two R groups on each silicone atom may represent the same group or different groups. Preferably the two R groups represent the same group. Suitable R groups include methyl, ethyl, propyl, phenyl, methylphenyl and phenylmethyl. The preferred silicones are polydimethyl siloxane, polydiethylsiloxane, and polydimethylphenylsiloxane. Polydimethylsiloxane is especially preferred.

Other silicones suitable for use in the present invention include the cyclic silicones. These materials have the formula:

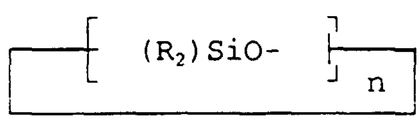

where n is 4 or 5 and R has the same meaning as in the structure of linear siloxanes.

The dimethyl cyclic siloxanes are volatile, and are thus present only temporarily after deposition. Volatile cyclic silicones are available under the trade name DOW CORNING 344 and 345 fluids from the Dow Corning Corporation.

Silicone used in this invention may well be a silicone homopolymer, although silicones may be modified by including copolymers, e.g. polyethers as is described in US Patent No. 3957970. Such copolymers tend to be more soluble than homopolymers of silicone.

Other materials may be included in compositions of this invention. Possibilities include colouring agents, opacifying agents, organic polymers, perfumes including deodorant perfumes, bactericidal agents to reduce the microflora on skin, antioxidants and other preservatives.

Organic polymers which may be present include cross-linked polyacrylates such as the Carbopol (TM) polymers available from Goodrich. These can function to increase viscosity or enhance stability of a composition. Polysaccharides are also well known as thickening agents and many are cellulose or cellulose derivatives.

Several methods can be employed to ensure that the rod micellar phase is present. One of these is perfume, the incorporation of perfume can thereby cause an increase in viscosity. Preferably in addition, or as an alternative, the increase in viscosity can be obtained by adding inorganic electrolyte. Thickening polymers may be included as mentioned above but will generally not be required.

Where electrolyte is present the level preferably exceeds 5% wt on product and, more preferably falls into the range 5-10% on product. The preferred electrolytes are alkali metal halides, preferably sodium chloride.

Compositions according to this invention are viscous emulsions with the silicone present as a disperse phase. Such emulsions can be formed by mixing the ingredients under conditions of high shear. However it may be preferred to introduce the silicone as a preformed emulsion so as to simplify mixing the composition.

It is preferred that the dispersed droplets of silicone oil have a mean particle size not greater than 2 μm, preferably the mean particle size is below 1.5 μm, most preferably in the range 0.1-1 μm.

<u>Example 1</u>

Detergent compositions were prepared containing:

| | % by weight |
|---|---|
| Sodium alkyl ether sulphate | 13.0 |
| Cocoamidopropylbetaine | 2.0 |
| Silicone | 2.0 |
| Jaguar C-13-S | 0.1 |
| Perfume | 1.0 |
| Sorbic acid | 0.37 |
| Trisodium citrate | 0.37 |
| Sodium chloride | see below |
| Water | balance to 100% |

The alkyl ether sulphate was sodium lauryl ether sulphate with average 3 ethylene oxide residues per molecule, available as Genopol ZRO (TM) from Hoechst.

The cocoamidopropylbetaine was Amonyl 380BA (available from Seppic).

As mentioned above Jaguar C-13-S is a cationic guar gum derivative with the CTFA designation guar hydroxypropyltrimonium chloride, available from Celanese-Stein Hall.

The silicone was added as 10% of a preformed emulsion BY22-026 from Toray Silicone Co Ltd comprising:

| | |
|---|---|
| Lauryl alcohol ethoxylate 2EO | 2% |
| Lauryl alcohol ethoxylate 21EO | 2% |
| Polydimethyl siloxane (60,000 cS) | 50% |
| Preservative | q.s. |
| Water | balance to 100% |

The silicone particles in this emulsion have a mean particle size of 0.4μm.

The compositions were made by mixing all the ingredients except sodium chloride in a paddle stirrer, and then mixing in the sodium chloride which led to an increase in viscosity of the composition.

Compositions were made containing various quantities of sodium chloride. Their viscosities were measured. The compositions were stored at 37°C and observed periodically to asses their stability. The results obtained are given below.

| Composition No. | 1 | 2 | 3 |
|---|---|---|---|
| wt% NaCl | 1.95 | 2.30 | 3.00 |
| Viscosity (Pa.sec) at 25°C, 10 $sec^{-1}$ | 4.8 | 7.5 | 11.0 |
| Storage at 20°C for 3 months | stable | stable | stable |
| Storage at 37°C for 3 months | slight separation of silicone | stable | stable |

Portions of the compositions were subjected to freeze-thaw cycling, alternating 12 hours at -10°C with and 12 hours at 20°C. After 6 cycles none of the compositions showed any instability.

Example 2

A number of compositions were prepared, with ingredients as set out in the following table. They were tested for stability by storing for 6 to 12 months at 25°C. None showed any instability.

| Ingredient | 1 | 2 | 3 | 4 | 5 | 6 |
|---|---|---|---|---|---|---|
| Sodium lauryl ether sulphate 3EO | 12 | 12 | 12 | 12 | 12 | 12 |
| Cocobetaine | 3 | 3 | 3 | 3 | 3 | 3 |
| NaCl | 1.5 | 1.5 | 1.5 | 1.5 | 1.5 | 1.5 |
| Jaguar C-13-S | 0.1 | 0 | 0.25 | 0 | 0.05 | 0 |
| Polymer JR400 | 0 | 0.1 | 0 | 0.25 | 0 | 0.05 |
| Silicone (BY22-026) | 5 | 5 | 5 | 5 | 5 | 5 |
| Perfume | 1 | 1 | 1 | 1 | 1 | 1 |
| Formalin | 0.1 | 0.1 | 0.1 | 0.1 | 0.1 | 0.1 |
| Water | balance to 100% | | | | | |
| Viscosity (Pa. sec at 10 $sec^{-1}$) | 10.5 | 10.5 | 10.2 | 10.0 | 9.5 | 10.0 |

The coconut betaine was of formula:

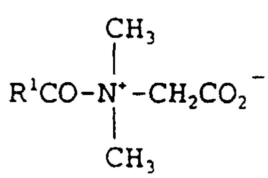

in which $R^1CO$- is a mixture of acyl groups derived from coconut. This betaine was Empigen (Registered Trade Mark) BB from Albright and Wilson.

The silicone oil was added as 10% of the preformed BY22-026 Emulsion from Toray Silicone Co Ltd.

Example 3

In this Example and those which follow, a number of tests were carried out by human volunteers. The experimental procedure employed was as follows:

The volunteer washed one forearm with a control shower gel containing neither silicone nor cationic polymer. This was carried out by wetting the arm and also the volunteer's free hand with warm water then using the free hand to lather the arm with 0.5 grams of the control shower gel, next rinsing for 10 seconds while rubbing with the free hand and then drying the arm with a paper towel.

After this pre-washing the volunteer washes the same forearm with a test product using the same procedure. When drying the forearm care is taken that the paper towel is drawn only once across a test area of the forearm.

10 minutes after drying the forearm the volunteer presses a strip of adhesive tape onto the area keeping it in place for 30 seconds using a spring loaded device bearing on a rubber bung to press the tape onto the skin with a repeatable pressure of $85g.cm^{-2}$. The adhesive tape employed was J-Lar Superclear (TM) tape having a width of 25mm. 5 or more such strips of tape are applied in this way one after another to the same area of skin.

In this test procedure silicone which has deposited on the skin will subsequently be transferred to the tape along with some of the outer layer of the volunteer's skin.

The amounts of silicone and skin adhering to the tape are determined by means of X-ray fluorescence spectroscopy. The tape strips are placed in an X-ray fluorescence spectrometer with the adhesive side facing the beam of this machine. A mask is applied over the tape to define a standardised area in the middle of the tape which is exposed to the X-ray beam. The sample chamber of the machine is placed under vacuum before making measurements and the spectrometer is then used to measure the quantities of silicone and sulphur. The sulphur is representative of the amount of skin which has transferred to the tape.

The amounts of silicone and sulphur observed with a clean piece of adhesive tape are subtracted from the experimental measurements. The experimental measurements for successive pieces of tape are added together and the cumulative totals of silicone and sulphur are expressed as a ratio of silicone to sulphur.

A shower gel contained the following:

| | % by weight |
|---|---|
| Sodium cocoyl isethionate | 9.0 |
| Coconut betaine | 6.0 |
| Silicone oil | 5.0 |
| Jaguar C-13-S | 0.1 |
| Formalin | 0.1 |
| Water | balance to 100% |

The sodium cocoyl isethionate was Fenopon (Registered Trade Mark) AC78 available from GAF Corporation. The coconut betaine was of Empigen.

The silicone was again added as 10% of the preformed emulsion BY22-026 from Toray Silicone Co Ltd.

The shower gel composition was made by mixing all the ingredients in a paddle stirrer. This led to an increase in viscosity of the mixture. After mixing the viscosity of the composition it was measured as 15,0 Pa.S at 10 $sec^{-1}$.

Silicone deposition by this composition was measured by the test procedure described above. It was compared with silicone deposition from an identical composition omitting the Jaguar C-13-S.

The cumulative results from 8 test strips were:

| | Total Si | Total S | Si/S Ratio |
|---|---|---|---|
| with Jaguar | 2.8 | 4.7 | 0.60 |

(continued)

| | Total Si | Total S | Si/S Ratio |
|---|---|---|---|
| without Jaguar | 0.4 | 4.4 | 0.09 |

Example 4

A shower gel contained the following:

| | % by weight |
|---|---|
| Sodium cocoyl isethionate | 5.0 |
| Coconut amidopropyl betaine | 8.0 |
| Sodium lauryl ether sulphate 3EO | 2.0 |
| Silicone oil | 5.0 |
| Jaguar C-13-S | 0.1 |
| Formalin | 0.1 |
| Perfume | 1.0 |
| Water | balance to 100% |

The coconut isethionate was Fenopon AC78 as in the previous Example.
The coconut amidopropyl betaine was of the formula:

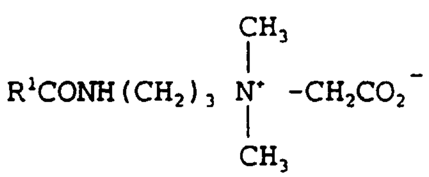

in which $R^1CO$- is a mixture of acyl groups derived from coconut. This was Rewoteric AMB 14 (TM) from Rewo.

The sodium lauryl ether sulphate was Empicol 0251 from Albright and Wilson.
The silicone was again BY22-026 emulsion.
Again the shower gel was made by mixing the ingredients, with the salt added last. Viscosity of the composition was measured as 15,000 centipoise at 10 $sec^{-1}$.
Silicone deposition was measured by the procedure described, and compared with that from an identical composition without the Jaguar C-13-S. The cumulative results from 8 test strips were:

| | Total Si | Total S | Si/S Ratio |
|---|---|---|---|
| with Jaguar | 2.0 | 4.9 | 0.41 |
| without Jaguar | 0.6 | 5.2 | 0.12 |

Example 5

The silicone deposition from compositions 1 and 2 of Example 2 was tested by the procedure given in Example 3 and compared with deposition from a similar composition without polymer. The cumulative results obtained from 7 test strips were:

| | Total Si | Total S | Si/S Ratio |
|---|---|---|---|
| with Jaguar | 3.7 | 4.4 | 0.84 |
| with JR400 | 4.2 | 5.3 | 0.79 |
| without Jaguar JR400 | 0.6 | 4.2 | 0.14 |

## Claims

**1.** A detergent composition in the form of an aqueous liquid or gel, a substantial part of which is in the rod-micellar phase, containing:

5 to 50% by weight of non-soap detergent,
0.01 to 5% by weight of cationic polymer, and
2.0 to 15% by weight of silicone,
where the detergent composition has a viscosity of at least 6.0 Pa.s (6000cP) at a shear rate of 10 $sec^{-1}$, the composition not including more than 0.5% by weight of structuring compounds in the form of esters or alcohols incorporating two alkyl or acyl groups with more than 20 carbon atoms in total, nor more than 0.5% by weight of Scleroglucan gums.

**2.** A shampoo in the form of an aqueous liquid or gel, a substantial part of which is in the rod-micellar phase, containing:

5 to 50% by weight of non-soap detergent,
0.01 to 5% by weight of cationic polymer, and
0.5 to 15% by weight of silicone,
where the detergent composition has a viscosity of at least 6.0 Pa.s (6000cP) at a shear rate of 10 $sec^{-1}$, the composition not including more than 0.5% by weight of structuring compounds in the form of esters on alcohols incorporating two alkyl or acyl groups with more than 20 carbon atoms in total, nor more than 0.5% by weight of Scleroglucan gums.

**3.** A composition according to claim 1 or 2 wherein the detergent comprises 30 to 100% by weight, based on the total surfactant, of anionic surfactant.

**4.** A composition according to claim 1, 2 or 3 wherein the detergent comprises 20 to 50% by weight, based on the total detergent, of a zwitterionic surfactant which has a hydrophilic head group containing both a quaternary nitrogen atom and at least one acid group.

**5.** A composition according to claim 4 wherein the zwitterionic detergent is of the general formula:

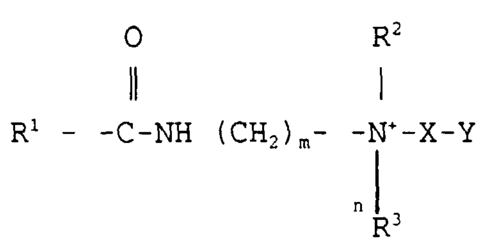

where $R^1$ is alkyl of alkenyl of 7 to 18 carbon atoms
$R^2$ and $R^3$ are each independently alkyl, hydroxyalkyl or carboxyalkyl of 1 to 3 carbon atoms
m is 2 to 4
n is 0 or 1.
X is alkylene of 1 to 3 carbon atoms optionally substituted with hydroxyl, and
Y is $-CO_2^-$ or $-SO_3^-$

6. A composition according to any one of the preceding claims containing 0.05% to 2% by weight of cationic polymer.

7. A composition according to any one of the preceding claims wherein the cationic polymer is selected from the group consisting of cationic cellulose ethers and cationic polygalactomannan gums.

8. A composition according to any one of the preceding claims wherein the silicone is a polysiloxane with the structure:

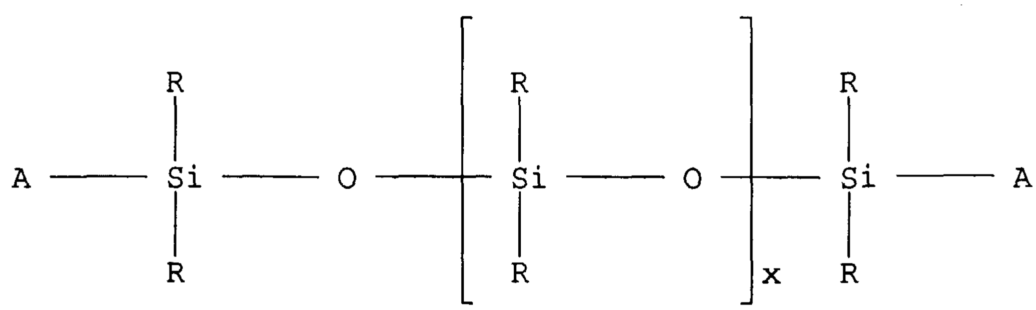

wherein R is alkyl or aryl, x is an integer from about 100 to about 2,400 and A represents groups which block the ends of the silicone chains.

9. A composition according to any one of the preceding claims containing 2% to 8% by weight of silicone.

10. A composition according to claim 8 containing 3.5 to 8% by weight of silicone oil.

11. A composition according to any one of the preceding claims wherein the silicone oil has a viscosity of at least 5 centistokes at 25°C.

12. A composition according to any one of the preceding claims which also contains inorganic electrolyte.

13. A composition according to any one of the preceding claims wherein the silicone oil is dispersed as droplets with particle size no larger than 2.0µm.

**Patentansprüche**

1. Eine Waschmittelzusammensetzung in der Form einer wässerigen Flüssigkeit oder eines Gels, wovon ein wesentlicher Teil in der Stab-micellaren Phase ist, enthaltend:

5 bis 50 Gewichtsprozent Nicht-Seife-Waschmittel,
0,01 bis 5 Gewichtsprozent kationisches Polymeres, und
2,0 bis 15 Gewichtsprozent Silicon,
worin die Waschmittelzusammensetzung eine Viskosität von zumindest 6,0 Pa.s (6000 cP) bei einer Schergeschwindigkeit von 10 $s^{-1}$ hat, die Zusammensetzung nicht mehr als 0,5 Gewichtsprozent Strukturierungsverbindungen in der Form von Estern oder Alkoholen enthält, einschliessend zwei Alkyl- oder Acylgruppen mit mehr als 20 Kohlenstoffatomen insgesamt, noch mehr als 0,5 Gewichtsprozent Scleroglucan-Gummis.

2. Ein Shampoo in der Form einer wässerigen Flüssigkeit oder eines Gels, wovon ein wesentlicher Teil in der Stabmicellaren Phase ist, enthaltend:

5 bis 50 Gewichtsprozent Nicht-Seife-Waschmittel,
0,01 bis 5 Gewichtsprozent kationisches Polymeres, und
0,5 bis 15 Gewichtsprozent Silicon,
worin die Waschmittelzusammensetzung eine Viskosität von zumindest 6,0 Pa.s (6000 cP) bei einer Schergeschwindigkeit von 10 $s^{-1}$ hat, die Zusammensetzung nicht mehr als 0,5 Gewichtsprozent Strukturierungsverbindungen in der Form von Estern oder Alkoholen enthält, einschliessend zwei Alkyl- oder Acylgruppen mit mehr als 20 Kohlenstoffatomen insgesamt, noch mehr als 0,5 Gewichtsprozent Scleroglucan-Gummis.

**3.** Eine Zusammensetzung nach Anspruch 1 oder 2, worin das Waschmittel 30 bis 100 Gewichtsprozent, basierend auf dem gesamten Tensid, an anionischem Tensid, umfaßt.

**4.** Eine Zusammensetzung nach Anspruch 1, 2 oder 3, worin das Waschmittel 20 bis 50 Gewichtsprozent, basierend auf dem gesamten Waschmittel, eines zwitterionischen Tensids umfaßt, welches eine hydrophile Kopfgruppe aufweist, enthaltend sowohl ein quaternäres Stickstoffatom und zumindest eine Säuregruppe.

**5.** Eine Zusammensetzung nach Anspruch 4, worin das zwitterionische Waschmittel die allgemeine Formel:

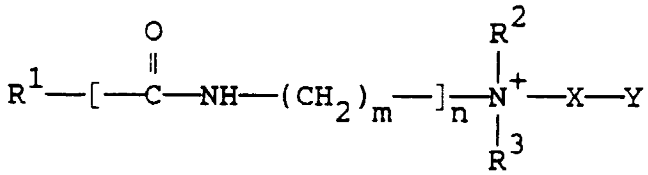

aufweist, worin $R^1$ Alkyl oder Alkenyl von 7 bis 18 Kohlenstoffatomen ist,
$R^2$ und $R^3$, jeder unabhängig, Alkyl, Hydroxyalkyl oder Carboxyalkyl von 1 bis 3 Kohlenstoffatomen sind,
m 2 bis 4 ist,
n 0 oder 1 ist,
X Alkylen von 1 bis 3 Kohlenstoffatomen ist, gegebenenfalls substituiert mit Hydroxyl, und
Y $-CO_2^-$ oder $-SO_3^-$ ist.

**6.** Eine Zusammensetzung nach irgendeinem der vorhergehenden Ansprüche, enthaltend 0,05 % bis 2 Gewichtsprozent kationisches Polymeres.

**7.** Eine Zusammensetzung nach irgendeinem der vorhergehenden Ansprüche, worin das kationische Polymere aus der Gruppe bestehend aus kationischen Celluloseethern und kationischen Polygalactomannan-Gummis ausgewählt ist.

**8.** Eine Zusammensetzung nach irgendeinem der vorhergehenden Ansprüche, worin das Silicon ein Polysiloxan mit der Struktur bedeutet:

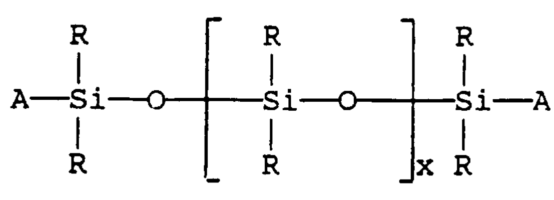

worin R Alkyl oder Aryl ist, x eine ganze Zahl von etwa 100 bis etwa 2400 ist und A Gruppen bedeutet, welche die Enden der Silicon-Ketten blockieren.

**9.** Eine Zusammensetzung nach irgendeinem der vorhergehenden Ansprüche, enthaltend 2 % bis 8 Gewichtsprozent Silicon.

**10.** Eine Zusammensetzung nach Anspruch 8, enthaltend 3,5 bis 8 Gewichtsprozent Siliconöl.

**11.** Eine Zusammensetzung nach irgendeinem der vorhergehenden Ansprüche, worin das Siliconöl eine Viskosität von zumindest 5 Centistoke bei 25°C hat.

**12.** Eine Zusammensetzung nach irgendeinem der vorhergehenden Ansprüche, welche auch anorganischen Elektrolyt enthält.

**13.** Eine Zusammensetzung nach irgendeinem der vorhergehenden Ansprüche, worin das Siliconöl als Tröpfchen mit Teilchengröße von nicht größer als 2,0 μm dispergiert ist.

**Revendications**

**1.** Composition détergente sous forme d'un liquide ou d'un gel aqueux, dont une partie sensible est en phase micellaire en bâtonnets, contenant :

5 à 50% en poids d'un détergent non savonneux,
0,01 à 5% en poids de polymère cationique, et
2,0 à 15% en poids de silicone,
la composition détergente ayant une viscosité d'au moins 6,0 Pa.s (6000 cP) à un taux de cisaillement de 10 $s^{-1}$, la composition ne comportant pas plus de 0,5% en poids de composés structurants sous forme d'esters ou d'alcools incorporant deux groupes alkyle ou acyle avec plus de 20 atomes de carbone au total, ni plus de 0,5% en poids de gommes SCLEROGLUCAN.

**2.** Shampooing sous forme d'un liquide ou d'un gel aqueux dont une partie sensible est en phase micellaire en bâtonnets contenant :

5 à 50% en poids d'un détergent non savonneux,
0,01 à 5% en poids de polymère cationique, et
2,0 à 15% en poids de silicone,
la composition détergente ayant une viscosité d'au moins 6,0 Pa.s (6000 cP) à un taux de cisaillement de 10 $s^{-1}$, la composition ne comportant pas plus de 0,5% en poids de composés structurants sous forme d'esters ou d'alcools incorporant deux groupes alkyle ou acyle avec plus de 20 atomes de carbone au total, ni plus de 0,5% en poids de qommes SCLEROGLUCAN.

**3.** Composition selon la revendication 1 ou 2, dans laquelle le détergent comprend 30 à 100% en poids, basé sur le tensioactif total, de tensioactif anionique.

**4.** Composition selon la revendication 1, 2 ou 3, dans laquelle le détergent comprend 20 à 50% en poids basé sur le détergent total d'un tensioactif zwitterionique qui a un groupe de tête hydrophile contenant à la fois un atome d'azote quaternaire et au moins un groupe acide.

**5.** Composition selon la revendication 4, dans laquelle le détergent zwitterionique est de formule générale :

$$R^1 - [-\overset{\overset{\displaystyle O}{\|}}{C}-NH\ (CH_2)_m-]_n-\underset{\underset{\displaystyle R^3}{|}}{\overset{\overset{\displaystyle R^2}{|}}{N^+}}-X-Y$$

dans laquelle $R^1$ est un alkyle ou un alcényle de 7 à 18 atomes de carbone, $R^2$ et $R^3$ sont chacun indépendamment un alkyle, un hydroxyalkyle ou un carboxyalkyle de 1 à 3 atomes de carbone,
m est 2 à 4

n est 0 ou 1
X est un alkylène de 1 à 3 atomes de carbone facultativement substitué avec un hydroxyle et
Y est $-CO_2^-$ ou $-SO_3^-$.

6. Composition selon l'une quelconque des revendications précédentes, contenant 0,05 à 2% en poids de polymère cationique.

7. Composition selon l'une quelconque des revendications précédentes, dans laquelle le polymère cationique est choisi parmi les éthers cellulosiques cationiques et les gommes de polygalactomannane cationiques.

8. Composition selon l'une quelconque des revendications précédentes dans laquelle la silicone est un polysiloxane de structure :

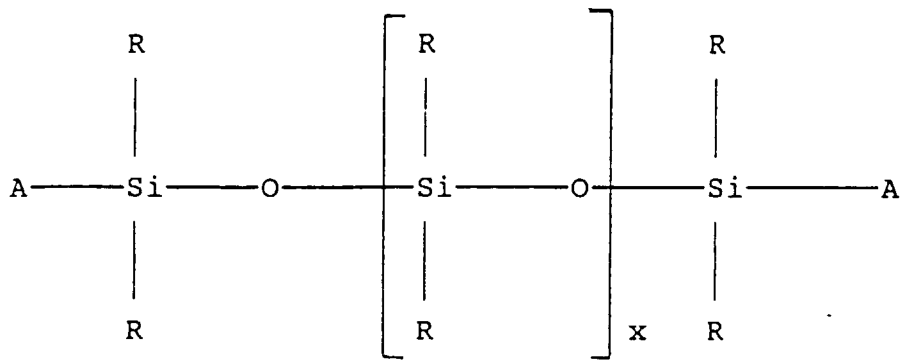

dans laquelle R est un alkyle ou un aryle, x est un nombre entier d'environ 100 à 2400 et A représente des groupes qui bloquent les extrémités des chaînes de silicone.

9. Composition selon l'une quelconque des revendications précédentes contenant 2 à 8% en poids de silicone.

10. Composition selon la revendication 8, contenant 3,5 à 8% en poids d'huile de silicone.

11. Composition selon l'une quelconque des revendications précédentes, dans laquelle l'huile de silicone a une viscosité d'au moins 5 centistokes à 25°C.

12. Composition selon l'une quelconque des revendications précédentes, qui contient également un électrolyte minéral.

13. Composition selon l'une quelconque des revendications précédentes, dans laquelle l'huile de silicone est dispersée sous forme de gouttelettes avec une granulométrie pas plus grande que 2,0 μm.